(19) Europäisches Patentamt
European Patent Office
Office européen des brevets

(11) Publication number: 0 031 991 B1

(12) **EUROPEAN PATENT SPECIFICATION**

(45) Date of publication of patent specification: 20.07.83

(21) Application number: 80303520.3

(22) Date of filing: 07.10.80

(51) Int. Cl.³: C 07 C 139/12, C 07 C 143/11, C 07 C 143/14, C 07 C 143/06, C 07 C 143/00//B01F17/00

(54) **Method of preparing propane sulfonates.**

(30) Priority: 23.11.79 US 96947

(43) Date of publication of application: 15.07.81 Bulletin 81/28

(45) Publication of the grant of the patent: 20.07.83 Bulletin 83/29

(84) Designated Contracting States: BE DE FR GB IT NL

(56) References cited:
DE - A - 2 748 722
DE - B - 1 173 906
GB - A - 995 376

JOURNAL OF THE AMERICAN OIL CHEMISTS' SOCIETY, Vol. 53, No. 1, January 1976 Champaign,
N. PARRIS ET AL: "XII. Alternate Syntheses of Surface Active Sulfobetaines" pages 60 to 63

(73) Proprietor: MOBIL OIL CORPORATION
150 East 42nd Street
New York New York 10017 (US)

(72) Inventor: Chen, Catherine Shuihua Hsia
102 Pinegrove Road
Berkeley Heights New Jersey 07922 (US)
Inventor: Williams, Albert Lloyd
9 Bay Berry Road, R.D. 2
Princeton New Jersey 08540 (US)
Inventor: Schmitt, Kirk Douglas
217 N. Main Street
Pennington New Jersey 08534 (US)

(74) Representative: West, Alan Harry et al,
Mobil Court 3 Clements Inn
London WC2A 2EB (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 031 991 B1

# Method of preparing propane sulfonates

This application is directed to an improved method of preparing propane sulfonates. Propane sulfonates are useful as surfactants or surface active agents; because they have high tolerance for brine they are especially useful in teritiary oil recovery processes.

Propane sulfonates of various amines and polyethoxylated alcohols are known surfactants. However, propane sulfonates of alcohols and thiols have, in the past, been prepared by reaction of alkali metal salts of the alcohols or thiols with propane sultone. This is a convenient high yield laboratory synthesis but is not desirable on a large scale for several reasons. First, the reaction requires multistep synthesis and purification of propane sultone; the propane sultone is expensive to purify and its overall yield of 80—90% limits the yield in the preparation of propane sulfonates. Second, propane sultone is a known carcinogen and processes involving its use must utilize expensive controls to minimize worker exposure and to ensure that no residues remain in waste products from the process.

A process for the preparation of propane sulfonates of tertiary amines is described in *J. Amer. Oil Chem. Soc., 53,* 60 (1976) but the process is said to produce excessive quantities of undesirable "iso-sulfonate" whose presence degrades the performance of the product. British Patent 1,566,655 teaches the preparation of ether sulfonates through the reaction of an alkyl compound with a bisulfite. In this process, sulfonate product is not initially added to the reaction mixture. Product yields of 82% and 81.6% are claimed. The process of the present invention utilizes a reaction mixture containing the sulfonate product and produces superior yields of up to 92%.

The reaction of bisulfites with simple olefins has been studied. Simple water-soluble olefins or olefins which can be made soluble by the addition of small amounts of alcohols can be converted in good yield to the desired products by dissolving all the reaction components in a single phase. We have found, however, that the allyl-heteroatom compounds do not behave this way. Conditions may be found in which all the reagents are dissolved in a single phase in alcohol and water and yet the conversion will not exceed 40 or 50%.

The early literature on the reaction of bisulfites with olefins indicated that S-alkylation leading to sulfonate products might be accompanied by a slight amount of O-alkylation leading to sulfites but even this slight formation was disputed and the literature for the last thirty years reveals that sulfites are not by-products in the sulfitation of olefins. We have found that sulfitation of allyl-hetero compounds in solvents which are supposed to be suitable for sulfitation to sulfonates may produce as much as 100% of the sulfite. This is all the more surprising because the literature teaches that solvent systems which produce high conversions of olefins will produce high yields of sulfonates.

We have now found that propane sulfonate derivatives of various alcohols, thiols and tertiary amines may be prepared by reacting the starting material with an allyl halide (or tosylate) to produce an olefinically unsaturated intermediate which itself is an allyl compound. This unsaturated intermediate is then reacted with a bisulfite salt to produce the final propane sulfonate product. This step of the reaction is carried out in the presence of a predetermined minimum amount of the final sulfonate product, the molar ratio of product to intermediate being at least 1:5.

The first step of this reaction, the preparation of the intermediate allyl ether, sulfide or amine has been described in the literature. The second step represents a new departure however, in that it permits the conversion of the allyl compound to the sulfonate product in good yields. The second step is carried out in the presence of water, using an alcohol to promote mutual dissolution of the reaction components.

The complete reaction sequence starting from hydroxy and thiol compounds can be represented as follows:

$$RXH + CH_2{=}CHCH_2Y \overset{\text{Base}}{=} RXCH_2CH{=}CH_2 + HY$$

$$RXCH_2CH{=}CH_2 + HMSO_3 = RXCH_2CH_2C_2SO_3M$$

where

R is a $C_6$ to $C_{16}$ alkyl, alkenyl or alkynyl or aryl of 6 to 20 carbon atoms.

R may also be an alkyl or aryl polyethoxy group selected from

$R'O - (CH_2CH_2O)_n-$ and 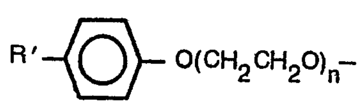

where

R' is a $C_6$—$C_{20}$ alkyl.

Y is halogen or tosylate

n is 1 to 13

M is alkali metal or ammonium
X is O or S.

In the first step the base binds the acid by-product HY to provide the driving force for the reaction.

When the starting material is a tertiary amino compound, the reaction proceeds in a similar way but in this case, the presence of the basic amino nitrogen provides basic functionality, obviating the need for added base. In the second step, the basic nitrogen provides an opportunity for internal salt formation, as shown below:

$$R_3N + CH_2{=}CHCH_2Y \; = \; R_3\overset{+}{N}CH_2CH{=}CH_2^{-}\cdot Y$$

$$R_3\overset{+}{N}CH_2CH{=}CH_2\overset{-}{Y} + HMSO_3 = R_3\overset{+}{N}CH_2CH_2CH_2SO_3^{-} + MY$$

where R, Y and M are as previously defined.

As mentioned above, the first step of the reaction has been described in the literature. The second step is carried out by addition of a buffered solution of the bisulfite to an aqueous alcoholic solution containing both the allyl compound and a certain minimum amount of desired final product. During this step, the reaction mixture should be stirred vigorously and a slow stream of air or oxygen passed through the reaction vessel. The rate of flow of air or oxygen is not critical. The minimum molar ratio of sulfonate product to allyl intermediate is 1:5. A lower, water-soluble alcohol, such as ethanol, may be used to promote dissolution of the reaction components. Tertiary-butyl alcohol, ethanol and 1-propanol are the preferred alcohols.

A convenient method to minimize the amount of final product which must be used is to charge the reaction vessel with only a portion of the allyl compound, and enought final product to make the minimum 1:5 ratio and then to add the remaining allyl compound while the bisulfite solution is added at a rate to ensure the ratio of final product to allyl compound in the vessel is always greater than 1:5.

The reactions are generally carried out under ambient conditions of temperature and pressure although higher pressures and temperatures may be used if desired.

The cation of the bisulfite, M, is any suitable metal or $NH_4^+$, although alkali metals, particularly sodium and lithium, are preferred.

The preferred allyl compounds are the chloride and the bromide.

A preferred class of sulfonate products for use as detergents, especially in oil recovery processes, are the polyoxyalkylene propane sulfonates. These are made according to the present invention by using a starting material containing a polyoxyalkylene group (R is one of the groups containing a polyoxyalkylene chain, above). These may be conveniently reacted with the allyl compound by refluxing the starting material with sodium in toluene until the sodium dissolves and then refluxing with the allyl halide or tosylate until the starting material has reacted. High pressure liquid chromatography (HLPC) may be used to indicate disappearance of the starting material.

The allyl ether (Allyl Ether A) used in the Examples below was produced in this way from polyethoxynonylphenol, as follows:

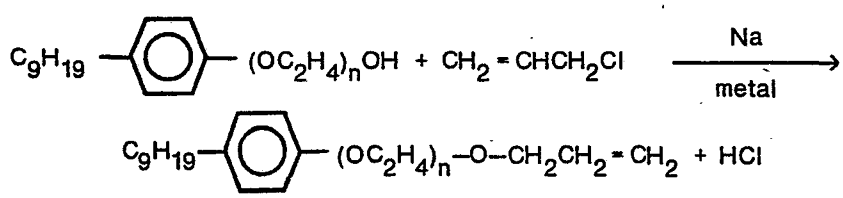


n = 4.2

The following Examples illustrate the effects of varying the reaction conditions described above.

Example 1

This Example 1 shows that high conversion of the allyl compound may be achieved without high yields. It also describes the analytical procedures which establish the structure of the sulfite product.

A one liter 4-neck flask equipped with a mechanical stirrer was charged with 120 ml. methanol, 75 ml. $H_2O$, 27 g of the sulfonate having the formula:

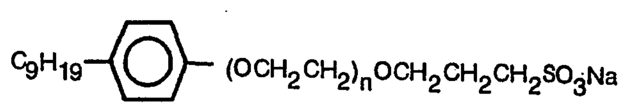

n = 4.2

and 66.9 g Allyl Ether A. A homogeneous solution resulted which was stirred at about 800—1000 RPM while air was passed through the flask at about 15 ml/min and a solution of 18.72 g $NaHSO_3$ and 11.34 g $Na_2SO_3$ in 69 ml $H_2O$ was added drowise over about one hour. Stirring was stopped occasionally and the reaction mixture analyzed by HPLC on a Water's Associates Micro-Bondapak C—18 (trademark) column using 0.005M $Bu_4N^+$ $H_2PO_4$— in 86% methanol — 14% $H_2$ as eluant. A UV detector set for 277 nm was used. Since each of the compounds has the same UV chromophore with maximum absorption at 277 nm the output of the detector could be translated directly into mole percent.

As the reaction proceeded the temperature rose exothermally about 5°C. Analysis by HPLC showed three materials with retention volumes 4.3 ml., 6.7 ml. and 34.3 ml. The second and third peaks had retention volumes identical to the sulfonate above and the allyl ether respectively. Within two hours of the start of the reaction HPLC analysis indicated 95% of the allyl ether had reacted but only 14—15% of this had been converted to the sulfonate. The rest had been converted into a sulfite (described below) whose structure was shown by $^{13}C$ NMR and chemical degradation.

The reaction was let stand overnight then filtered from precipitated inorganic salts, evaporated on a rotary evaporator and a $^{13}C$ NMR obtained. In addition to the peaks expected for the sulfonate (by comparison to a spectrum of authentic sulfonate) two additional peaks due to one carbon each were seen at 62.9 ppm and 25.1 ppm. These are assigned to the $NaO_2S—O—CH_2—CH_2—$ carbons of a sulfite having the formula:

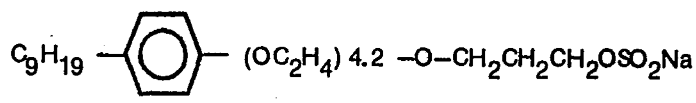

This material was shown to be the sulfite by heating the reaction product at 60°C at 7 Pa for four hours. A gas was collected in a dry ice trap. Infrared analysis showed this gas to be sulfur dioxide. Carbon-13 NMR of the remaining material in moist $CDCl_3$ shows the peak at 62.9 ppm to be gone and a new peak at 57.4 ppm to have appeared. This new chemical shift is identical to that of 3-methoxy-1-propanol. These results are summarized by the following reaction:

(4)
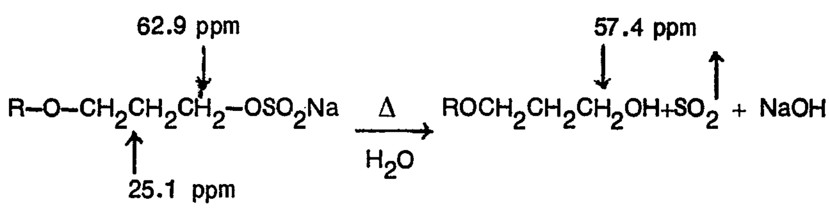


Example 2

The same reaction as Example 1 was carried out with ethanol substituted for methanol. After three hours the conversion of allyl ether was 99% and the yield of sulfonate was not 14% as in Example 1 but 92%.

Example 3

The same reaction as Example 1 was carried out but 1-propanol was substituted for methanol. The conversion after three hours was 94% and the yield 85%.

Example 4

The same reaction as Example 1 was carried out but 2-propanol was substituted for methanol. The conversion after three hours was 99% and the yield 84%.

Example 5

The same reaction as Example 1 was carried out but water was substituted for methanol. The conversion after three hours was 96% and the yield 3%.

Example 6

The same reaction as Example 2 was carried out but only 5.67 g $Na_2SO_3$ were used. The conversion after three hours was 90% and the yield 78%.

Example 7

The reaction of Example 2 was repeated but no sulfonate was added initially. A homogeneous solution did not result. After three hours there was no reaction.

Example 8

The reaction of Example 2 was repeated but 15 g sulfonate were used. The ratio of sulfonate to allyl compound was 0.189. The reaction mixture was homogeneous. After three hours the conversion was 87% and the yield 72%.

Example 9

The reaction of Example 2 was repeated but 9 g sulfonate were used. The reaction mixture was not homogeneous. The ratio of sulfonate to allyl compound was 0.113. After three hours the conversion was 40% and the yield 3%.

Example 10

The reaction of Example 2 was repeated but the $NaHSO_3$ and $Na_2SO_3$ were added as solids all at once to the homogeneous solution already containing the 69 ml $H_2O$. The conversion after three hours was 71% and the yield 6%.

Example 11

The reaction of Example 2 was repeated but tertiary-butyl alcohol was substituted for methanol. The conversion after three hours was 99% and the yield 86%.

Example 12

The reaction was carried out as in Example 3, but 36 g of heptaethoxydinonylphenol was used to prepare the allyl ether starting material, of which 68.6 g were used. The conversion after three hours was 97% and the yield was 90%.

The Examples show that the reactants and reaction conditions are important in achieving the desired product in high conversion and high yield. Note, for example, the yield of the desired product in Example 1 in comparison to that for Examples 2, 3 and 4. Note also that when water, as in Example 5, was substituted for the alcoholic reactant, although conversion was 96% the yield was only 3%. When the reaction mixture was not homogeneous as in Example 9, the conversion was only 40% and the yield only 3%.

**Claims**

1. A method of preparing propane sulfonates from allyl halides or tosylates which comprises reacting the allyl halide or tosylate with a hydroxyl, thiol or tertiary amino compound and reacting the resultant intermediate allyl product with a bisulfite salt characterised by reacting said intermediate in the presence of preformed propane sulfonate product, the ratio of preformed propane sulfonate product to the resultant intermediate allyl product being at least 1:5, in an aqueous alcoholic solution the alcohol of which has at least two carbon atoms.

2. A method according to claim 1 in which a first portion of the allyl compound is added to the reaction mixture and then sufficient final sulfonate product is added thereto to maintain the minimum 1 to 5 ratio, after which additional allyl compound is added at a rate which ensures the ratio will be greater than 1:5.

3. A method according to claim 1 or 2 in which the bisulfite is lithium or sodium bisulfite.

4. A method according to claim 1 or 2 in which the bisulfite is sodium bisulfite.

5. A method according to any of claims 1 to 4 in which the allyl halide is allyl chloride or allyl bromide.

6. A method according to any of claims 1 to 5 in which the final sulfonate product is

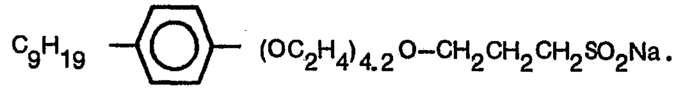

7. A method according to any of claims 1 to 6 in which the intermediate allyl compound is reacted with the bisulfite by the addition of a buffered solution of a suitable amount of the bisulfite to an aqueous alcoholic solution containing the allyl compound and the minimum amount of the final sulfonate product while passing a stream of air or oxygen through the reaction mixture.

8. A method according to claim 7 in which the alcohol is t-butanol, ethanol, 2-propanol or 1-propanol.

**Patentansprüche**

1. Verfahren zur Herstellung von Propansulfonaten aus Allylhalogeniden oder -tosylaten, das die Umsetzung des Allylhalogenids oder -tosylats mit einer Hydroxyl-, Thiol- oder tertiären Amin-Verbindung und die Umsetzung des Erhaltenen Allyl-Zwischenprodukts mit einem Bisulfitsalz umfaßt, gekennzeichnet durch Umsetzung des genannten Zwischenprodukts in Gegenwart eines bereits gebildeten Propansulfonat-Produkts in einer wässrigen alkoholischen Lösung, deren Alkohol wenigstens 2 Kohlenstoffatome aufweist, wobei das Verhältnis des bereits gebildeten Propansulfonat-Produkts zu dem erhaltenen Allyl-Zwischenprodukt wenigstens 1:5 beträgt.

2. Verfahren nach Anspruch 1, bei dem eine erste Teilmenge der Allylverbindung zu der Reaktionsmischung zugesetzt wird und danach ausreichend viel Sulfonat-Endprodukt zugesetzt wird, um das Mindestverhältnis von 1:5 aufrechtzuerhalten, wonach weitere Allylverbindung mit einer Geschwindigkeit zugesetzt wird, die sicherstellt, daß das Verhältnis größer als 1:5 ist.

3. Verfahren nach Anspruch 1 oder 2, bei dem das Bisulfit Lithium- oder Natrium-bisulfit ist.

4. Verfahren nach Anspruch 1 oder 2, bei dem das Bisulfit Natrium-bisulfit ist.

5. Verfahren nach einem der Ansprüche 1 bis 4, bei dem das Allylhalogenid Allylchlorid oder Allylbromid ist.

6. Verfahren nach einem der Ansprüche 1 bis 5, bei dem das sulfonierte Endprodukt

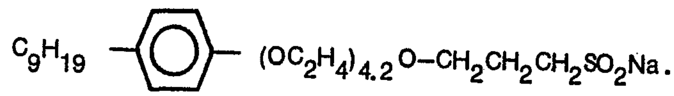

ist.

7. Verfahren nach einem der Ansprüche 1 bis 6, bei dem die Allyl-Zwischenverbindung mit dem Bisulfit durch die Zugabe einer gepufferten Lösung einer geeigneten Menge des Bisulfits zu einer wäßrigen alkoholischen Lösung umgesetzt wird, die die Allyl-Verbindung und die Mindestmenge des sulfonierten Endprodukts enthält, während ein Strom von Luft oder Sauerstoff durch die Reaktionsmischung geleitet wird.

8. Verfahren nach Anspruch 7, in dem der Alkohol t-Butanol, Ethanol, 2-Propanol oder 1-Propanol ist.

**Revendications**

1. Procédé de préparation de propanesulfonates à partir d'halogénures ou de tosylates d'allyle qui comprend la réaction de l'halogénure ou du tosylate d'allyle avec un composé hydroxylé, une amine tertiaire ou un thiol, puis la réaction du produit allylique intermédiaire résultant avec un sel bisulfite, caractérisé en ce que l'on fait réagir ledit intermédiaire en présence de propanesulfonate préformé, le rapport propanesulfonate préformé/produit allylique intermédiaire résultant étant d'au moins 1/5, dans une solution alcoolique aqueuse dont l'alcool contient au moins 2 atomes de carbone.

2. Procédé selon la revendication 1, caractérisé en ce qu'une première portion du composé allylique est ajoutée au mélange réactionnel et l'on y ajoute ensuite suffisamment de sulfonate final pour maintenir le rapport minimal 1/5, après quoi on ajoute le composé allylique supplémentaire à une vitesse telle que le rapport soit supérieur à 1/5.

3. Procédé selon la revendication 1 ou 2, caractérisé en ce que le bisulfite est le bisulfite de lithium ou de sodium.

4. Procédé selon la revendication 1 ou 2, caractérisé en ce que le bisulfite est le bisulfite de sodium.

5. Procédé selon l'une quelconque des revendications 1 à 4, caractérisé en ce que l'halogénure d'allyle est le chlorure d'allyle ou le bromure d'allyle.

6. Procédé selon l'une quelconque des revendications 1 à 5, caractérisé en ce que le sulfonate final est:

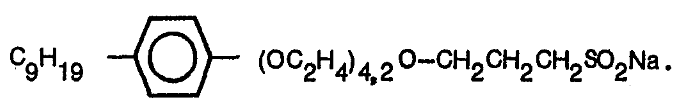

7. Procédé selon l'une quelconque des revendications 1 à 6, caractérisé en ce que l'on fait réagir le composé allylique intermédiaire avec le bisulfite en ajoutant une solution tamponnée d'une quantité appropriée du bisulfite à une solution alcoolique aqueuse contenant le composé allylique et la quantité minimale du sulfonate final, tout en faisant passer un courant d'air ou d'oxygène dans le mélange réactionnel.

8. Procédé selon la revendication 7, caractérisé en ce que l'alcool est le t-butanol, l'éthanol, le propanol-2 ou le propanol-1.